# EUROPEAN PATENT APPLICATION

(11) **EP 4 431 120 A1**
(43) Date of publication of application: **18.09.2024**
(21) Application number: 22892186.2
(22) Date of filing: 09.11.2022
(51) Int. Cl.: A61L 2/10, A61L 2/16, A61L 9/16, A41D 13/11, B01D 35/28, A62B 18/08

(54) **GERMICIDAL DEVICE FOR AIR AND FOR LIQUIDS**

(30) Priority: 09.11.2021 ES 202132213 U; 16.11.2021 ES 202132279 U
(71) Applicant: Andarias García, José Vicente, 13500 Puertollano (Ciudad Real) (ES)
(72) Inventor: Andarias García, José Vicente, 13500 Puertollano (Ciudad Real) (ES)
(74) Representative: De Castro Hermida, José Luis
(86) International application number: PCT/ES2022/070717
(87) International publication number: WO 2023/084139

(57) **Abstract**

Disclosed is a germicidal device for air and for liquids, characterised in that it has five different operation modes, which comprises a series of particle-retaining filters (2, 14, 22, 25, 35) made of activated carbon and silver-ion fabric and through which a current of fluid passes at different pressures, propelled by motor-turbines (16, 17) towards labyrinth plates (5, 6). The plates surround a refrigeration compartment (9) containing emitters (7) of germicidal radiation, which refrigerate and purify gases such as ozone, preventing the consumption thereof and allowing same to be used as a germicidal reinforcement in the system. Purified air to be inhaled and exhaled air are carried through tubes (31, 32) between the device and a mask worn by the user, which are attached to the neck or head by means of retentive supports (37, 39, 40, 44, 45).

## Description

### OBJECT OF THE INVENTION

As the title of the invention establishes, the object of the present invention relates to a germicidal device for air and for liquids, that is, it is a device that allows purifying both the air that the user inhales and the air that the user exhales, separately or at the same time, as well as purifying liquids. It is an innovation that provides previously unknown advantages among current techniques.

### FIELD OF APPLICATION OF THE INVENTION

The present invention is comprised in the industrial sector of personal well-being health and hygienic-sanitary treatments dedicated to the manufacture and use of sterilising equipment and services, as it is a sterilising device for fluids for consumption, based on the use of ultraviolet light with the possibility of adding biocides to the device.

The present invention relates to a portable personal device that helps purify both liquids to drink and air that is inhaled and exhaled, sterilising them from harmful germs and eliminating particles, advantages and characteristics that are described in detail below.

### BACKGROUND OF THE INVENTION

There are devices similar to the one described in the present invention that reflect the state of the art related to same, with different uses and methods to purify the air at an industrial and personal level, but none that does so at a personal or individual level efficiently in real time, which can be intercalated with and/or interconnected to other devices already implemented to further improve air quality.

The ability of ultraviolet light energy to destroy or neutralise microorganisms (bacteria, viruses, fungi, etc.) which, when subjected to this irradiation in a sufficient quantity and time, cause them to receive a lethal dose, that is, the product of light intensity times exposure time reaches a predetermined value depending on the type of germ to be combated, is well known.

The different germicidal wavelengths are between 200 nm and 280 nm and it is scientifically proven that UV-C radiation (short wavelength ultraviolet light) at 253.7 nm is the one that causes the greatest effectiveness in destroying the DNA of the cells of microorganisms, preventing their growth and reproduction.

The device object of the following invention is specifically designed to irradiate microorganisms with the necessary power and time, to destroy the vast majority of them with emitters of germicidal radiation which, in this case, for illustrative and non-limiting purposes, would be UV light (ultraviolet light), without adding any chemicals as a result of its exclusive gallery system, combined in a portable and lightweight device, with enough autonomy for several hours powered by removable and quickly interchangeable rechargeable batteries, and which further allows the possibility of charging other external devices such as tablets or telephones.

References of some of them are indicated below:
Document ES1070890U relates to an air sterilising and filtering device that incorporates its own mask.
Document ES1247195U relates to an inertial filter which has a structure where UVC LEDS are housed and retains particles and microorganisms.
Document ES1247683U relates to a mask that houses LEDS in the same filter.
ES1250689U, ES2182294T3 and ES2099472T3, germicides by emission of ultraviolet radiation, relate to a volumetric device that filters and sterilises large volumes of air.

Moreover, it should be noted that the applicant is unaware of the existence of any other similar device that presents technical and structural features identical or similar to those presented and claimed herein.

### DESCRIPTION OF THE INVENTION

The object of the present invention relates to a germicidal device for air and for liquids, the purpose of which is for users to have a compact, personal, portable, light, autonomous and rechargeable germicidal device, which combines in a single device what is estimated to be the most efficient, light and cost-effective way to render fluids inert to be manufactured, since it has several different applications as it can be used as a sterilising respirator for air and as a steriliser for liquids, comprising several operation modes selected by the user, where another of its benefits is that due to its design it does not require any chemicals, nor does it add odour or flavour to the fluid to be consumed.

The germicidal device for air and for liquids comprises accessories for retaining the mask and components that adapt the device to the user's physiognomy, which can be used together or separately interchangeably and which allow alleviating ear pain derived from rubber bands and anchor straps for the masks when they are fastened to them.

According to the different operation modes and paths for the fluids, the device is capable of treating the air adjacent to a healthy user who is going to inhale by rendering it inert before entering their lungs (inhalation mode), the air that a sick person has exhaled after exiting their lungs (exhalation mode) or both flows simultaneously (mixed mode), making the air around other users who do not have this device safe, thus preventing them from being contaminated with it, becoming infected and falling ill, thus solving one of several of the current problems in health and coexistence issues, such as the quality of the air at a biological level that is breathed in, where all that is known today is the growing risk that is faced, where said quality must be as inert as possible.

This device also allows improvement of the biological quality of water due to its scarcity, where inhabitants, campers or mountaineers who are in remote areas or isolated from the aquifer network where water purification is non-existent and must be supplied from streams, reservoirs or wells, can have completely safe water for their consumption.

The device is completely autonomous with rechargeable batteries and can be powered externally with electrical energy with a power source, and is also capable of powering other external devices through electronic ports such as USB.

The described device is designed with a small size, being a compact device that makes it lightweight, light and portable, taking up minimal space, which can be transported by users individually, this device being ideal for use in places where the mandatory proximity of people is inevitable and there is a need to maintain minimum distances even though there is a serious risk of infection, allowing them to be close to each other in complete safety.

It can be left installed in a fixed and hidden place in small spaces, providing extra safety with a jet of purified air to the user's face and/or mask or used to collect and treat the air exhaled by the user to disinfect it, and can be installed, for example, under a table where several diners will be sitting, desks in offices, hospital beds, seat backs of trains, ships, buses, subways, taxis, meeting places, workplaces or where even weight is an important factor to be taken into account, such as in airplanes.

The device itself is an electrical germicidal apparatus that biologically treats and renders viruses and bacteria of any type of fluid inert by means of the germicidal emission of different technologies, although for the description of the present application, as a non-limiting example, ultraviolet light emitters will be used, and they can be of various wavelength spectra between 100 nm and 400 nm, the most effective according to scientific studies being 254 nm, which acts directly on the DNA structure of microorganisms, breaking and damaging it, preventing the growth and reproduction thereof, with some lengths generating ozone, which contribute to the germicidal power although they constitute a risk to the user's health if breathed, a problem for which a technical solution is provided in more detail below.

The device treats the fluid, making it enter and exit through different inlets and outlets, depending on the operation mode, forced by the action of several turbine motors with variable pitch and speed that drive or expel it until it passes through the device from one end to the other, being filtered before entering, during the process and at the outlet of the device, by a battery of filters strategically placed to eliminate suspended particles, dust, allergens, viruses and bacteria, where said fluids are guided through a network of ducts and galleries. arranged in independent and isolated channels, which make them pass systematically around the germicidal source, forced by impulse and suction turbines.

In one mode of use, the germicidal device for air and for liquids provides a positive, continuous and purified flow of air rendered inert to the user's face or mask, thus preventing pockets of CO2 exhaled by the user from accumulating therein and being breathed in again.

In other modes of use, it is capable of collecting 100% of the air exhaled by the user and can be rendered inert, thus preventing it from being expelled into the atmosphere and contaminating other users.

The device has several independent circuits for rendering fluids inert and a separate and independent circuit intended for refrigerating the germicidal emitting device, since the prolonged use of certain light emitters generates heat that needs to be discharged to guarantee the durability thereof and which can further transfer it to the rest of the germicidal channels and in turn to the stream of fluid, as well as generate ozone and other poisonous gases that irritate the mucous membranes and lungs, so the heat and toxic gases are expelled through another independent outlet, thus preventing them from being breathed in by the user, mainly when the user inhales, since for the rest of the modes of use, the device makes these channels communicate with each other and takes advantage of these gases as a complement since ozone is also germicidal, thus increasing the disinfecting power of the device and multiplying the effect of the germicidal emitter system, so this design adds two more advantages to the device with respect to other devices, where users can breathe hot air with ozone.

The device's emitters of germicidal radiation do not lose effectiveness because the air introduced is free of any dust particle as it has already been filtered previously, and all microorganisms are irradiated and neutralised.

For the disinfection of liquids, the device uses all the available channels in addition to another extra reserve housed in another compartment, where chlorination tablets and/or biocides can furthermore be added, thus increasing the germicidal power in liquids that may contain a high load of microorganisms.

In pandemic situations, a mask must be worn as a barrier against viruses, where there are inevitable situations in which it must be removed in the presence of other people, such as while eating or drinking, and for this purpose, the germicidal device for air and for liquids comprises accessories that act as a complement, improving the user's well-being. To do this, the device is accompanied by retentive accessories for retaining the mask on the user's face, in addition to a tube and a diffuser that carry the air rendered inert to the user's face and/or the mask, the external volume of which is acquired depending on the airflow that the device sends to the user's face or mask.

The tube and diffuser can be moulded to personalise and ergonomically adapt to the user, facilitating comfort of use. For these elements to remain in the position desired by the user, the device is accompanied by anchoring accessories for anchoring them to the user's physiognomy, enabling the user to remove the mask at specific times such as when eating, without having to risk being infected by being next to other diners, as a result of the continuous and positive pressure of an airflow that will reach the user from the device through the tube and diffuser.

The fastening elements are intended to retain the mask on the user's face, thus avoiding the bending of the ears and the pain caused by the rubber bands and retaining straps for retaining said mask on the user, making it more comfortable to wear it.

### BRIEF DESCRIPTION OF THE DRAWINGS

To complete the description provided herein, and for the purpose of helping to make the features of the invention more readily understandable, this description is accompanied by figures constituting an integral part of the same, which by way of illustration and not limitation represents the following:

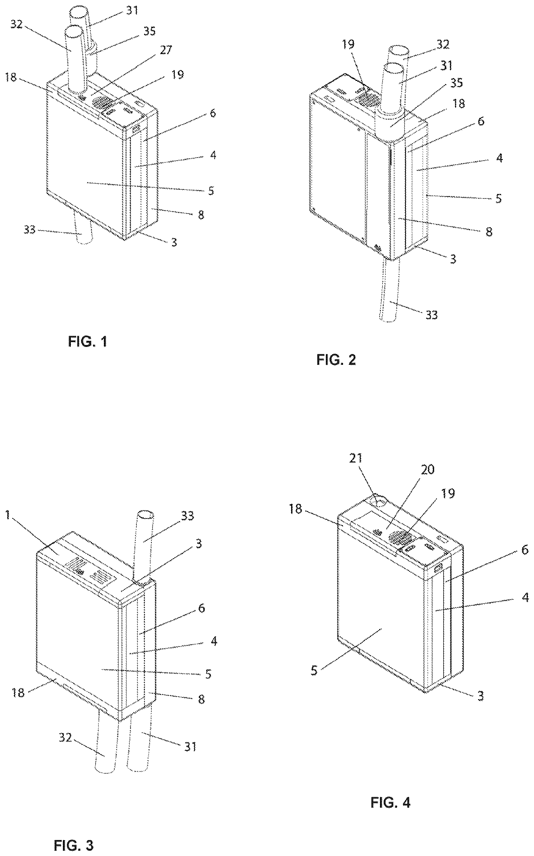

Figure 1 is a perspective representation of the germicidal device for air and for liquids, seen from the front.
Figure 2 is a perspective representation of the germicidal device for air and for liquids, seen from the rear.
Figure 3 is a perspective representation of the germicidal device for air and for liquids, seen from the front and bottom.
Figure 4 is a perspective representation of the germicidal device for air and for liquids, seen from the front and top, showing the upper cover (20) in the top portion, without showing the main tube (31), the secondary tube (32) and the auxiliary tube (33).

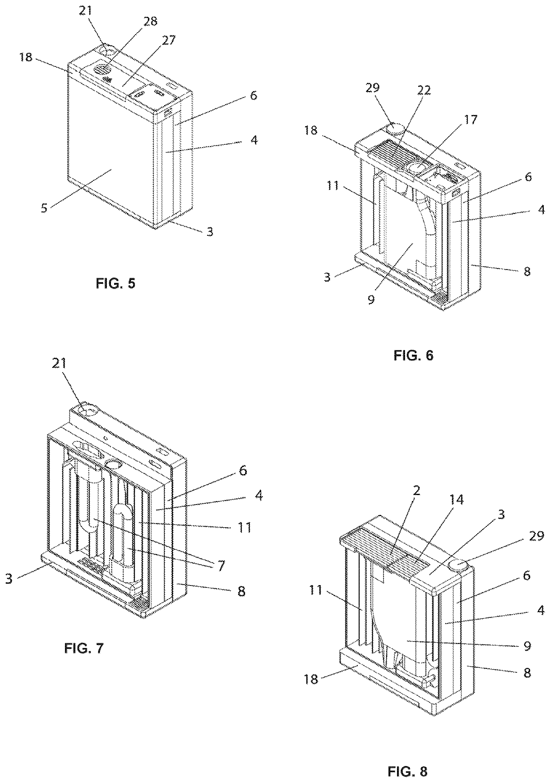

Figure 5 is a perspective representation of the germicidal device for air and for liquids, seen from the front and top, showing the interchangeable cover (27) in the top portion, without showing the main tube (31), the secondary tube (32) and the auxiliary tube (33).
Figure 6 is a perspective representation of the inside of the germicidal device for air and for liquids, seen from the front and top, hiding the primary labyrinth plate (5), as well as the upper cover (20) and the interchangeable cover (27), which allows seeing the inside thereof.
Figure 7 is a perspective representation of the inside of the germicidal device for air and for liquids, seen from the front and top, hiding the primary labyrinth plate (5), as well as the upper cover (20), the interchangeable cover (27), the refrigerator compartment (9) and the upper plate (18), which allows seeing the inside thereof.
Figure 8 is a perspective representation of the inside of the germicidal device for air and for liquids, seen from the front and bottom, hiding the primary labyrinth plate (5) as well as the cover (1), allowing the inside thereof to be seen.

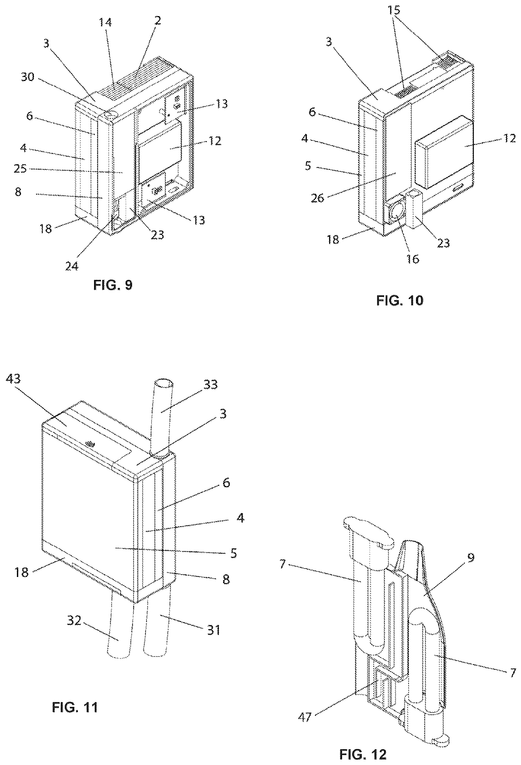

Figure 9 is a perspective representation of the inside of the germicidal device for air and for liquids, seen from the rear and bottom, hiding portions of the container plate (8) and the cover (1), allowing the inside thereof to be seen.
Figure 10 is a perspective representation of the inside of the germicidal device for air and for liquids, seen from the rear and bottom, hiding the container plate (8), the cover (1), the auxiliary cover (43), the main filter (2), the secondary filter (14) and the water filter (25), allowing the inside thereof to be seen.
Figure 11 is a perspective representation of the germicidal device for air and for liquids, seen from the front and bottom with the auxiliary cover (43).
Figure 12 is a perspective sectional representation of the refrigerator compartment (9) where the refrigerating perimeter walls (47) and the emitters (7) of germicidal radiation are shown in detail.

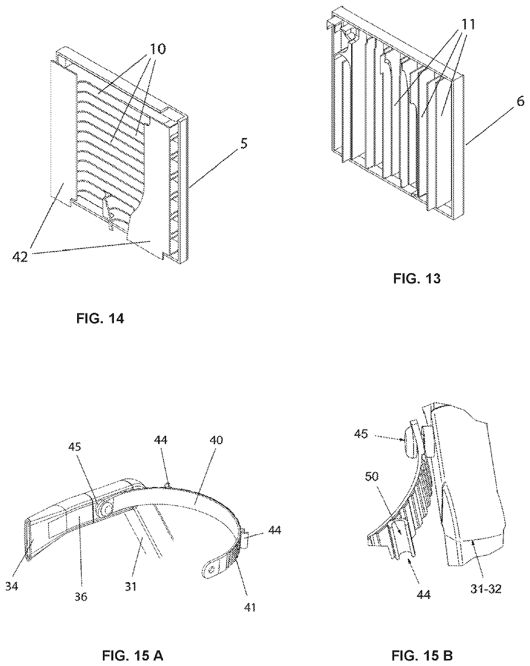

Figure 13 is a perspective representation of the secondary labyrinth plate (6) where the primary perimeter walls (11) are shown in detail.
Figure 14 is a perspective representation of the primary labyrinth plate (5) where the primary perimeter walls (10) and the separator (42) are shown in detail.
Figure 15A is a perspective and detailed representation of the accessories that act as a complement and are for retention of the user's mask, showing an embodiment where the strap (40), the support (44) and the connection (45) are used.
Figure 15B is a perspective and detailed representation of the accessories that act as a complement and are for retention of the user's mask, showing an embodiment where the connection (45), a section of the main tube (31) or of the secondary tube (32) interchangeably and the support (44) that accommodates the containment area (50) that retains the rubber bands or straps of the masks can be seen.

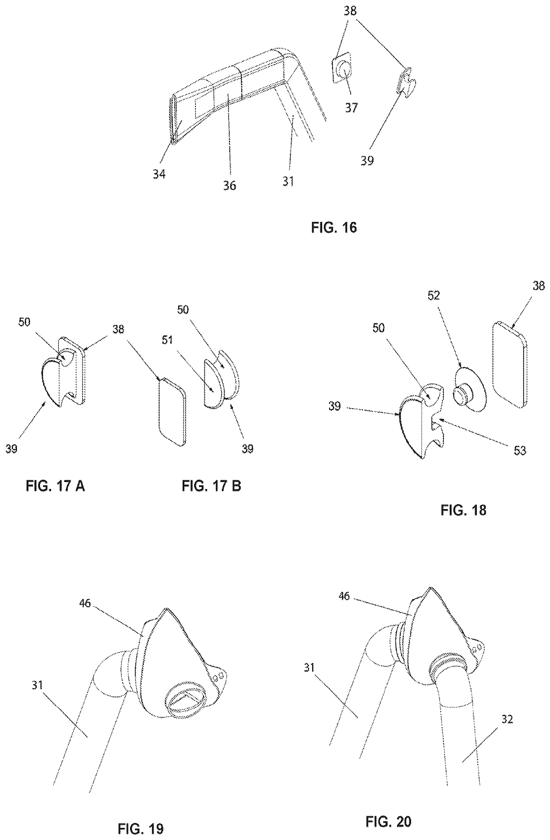

Figure 16 is a detailed perspective representation of the accessories that act as a complement and are for retention of the user's mask, showing an embodiment where the connectors (37) with the surfaces (38) and the anchors (39) are used.
Figure 17A is a perspective representation of the mask accessory in an embodiment with the anchors (39), the containment area (50) of the straps of the masks and the surface (38).
Figure 17B is a perspective representation of the mask accessory in an embodiment with the anchors (39), the containment area (50) of the straps of the masks and the surface (51) for connecting to the surface. (38).
Figure 18 is a perspective and sectional representation of the mask accessory in one embodiment, where the surface (38) connects the anchor (39) through the connector (52), configured to be housed in the cavity (53) that is in the anchor (39).
Figure 19 is a detailed perspective representation of the accessories that act as a complement and are for retention of the user's mask, showing an embodiment where the main tube (31) is directly connected to a mask (46).
Figure 20 is a detailed perspective representation of the accessories that act as a complement and are for retention of the user's mask, showing an embodiment where the main tube (31) and the secondary tube (32) are directly connected to a mask ( 46).

### PREFERRED EMBODIMENT OF THE INVENTION

In view of the figures, a device is described below that houses five different operation modes, which are INHALATION MODE, EXHALATION MODE, DUAL MODE, LIQUID MODE and MIXED MODE.

According to a preferred, although non-limiting, embodiment of the proposed invention, the germicidal device for air and for liquids, as seen in the figures, in INHALATION MODE is configured so that when the user only needs to inhale purified air, the fluid enters the device naturally or forcefully by means of a main motor-turbine-pump (16), through the removable and interchangeable cover (1), then passing through and being filtered by a main anti-particle and anti-microorganism filter (2), which is housed inside a plate (3) comprising several compartments and a plurality of holes through which the air passes to an intermediate plate (4), which internally accommodates the primary labyrinth plate (5) and the secondary labyrinth plate (6) that surround a plurality of emitters (7) of germicidal radiation that are in turn inside a refrigerator compartment (9) configured to refrigerate the emitters (7) of germicidal radiation, entrain and expel to the outside the possible emissions of ozone or other gases produced by said germicidal emitters (7).

Once the fluid enters the intermediate plate (4), it is then directed towards the primary labyrinth plate (5), which consists of a plurality of channels and primary perimeter walls (10) that may have irregularities on their surface which are configured to produce turbulence and facilitate germicidal treatment, made of a transparent material and which, due to their design and arrangement, guide and slow down the volume of flow of the fluid as it passes through same for the necessary and sufficient time, passing it multiple times around the emitters (7) of germicidal radiation, neutralising any microorganism that the fluid may contain, configured to emit radiation at any wavelength, not limited to ultraviolet light, emitting radiation of any other type that has germicidal and/or disinfecting power.

At the end of the path of the primary labyrinth plate (5), the fluid enters the secondary labyrinth plate (6), which comprises a plurality of channels and transparent secondary perimeter walls (11) which, due to their design and arrangement, guide and slow down the volume of flow of the fluid as it passes through same for the necessary and sufficient time, passing it multiple times around the emitters (7) of germicidal radiation, and likewise said secondary perimeter walls (11) may have irregularities on their surface which are configured to produce turbulence and facilitate germicidal treatment. Both the primary labyrinth plate (5) and the secondary labyrinth plate (6) are separated by means of a separator (42) configured to separate the labyrinth channels into two differentiated spaces, and it may be transparent.

The air exits through the container plate (8) to reach an encapsulation (35), the main tube (31) and a diffuser (34) configured to send the fluid towards the user's mask through a set of accessories explained later.

Moreover, the ambient air enters the refrigerating circuit of the emitters (7) of germicidal radiation forced by a secondary motor-turbine (17) through a removable and interchangeable cover (1), then passing through and being filtered by a secondary anti-particle and anti-microorganism filter (14), which is housed inside the plate (3) comprising several compartments with a plurality of openings (15) through which it continues to the intermediate plate (4). Said fluid passes through the refrigeration compartment (9) to exit at one end, passing through the intermediate plate (4), to enter an upper plate (18) with several compartments, where the secondary motor-turbine (17), which is configured to suck the fluid coming from the refrigerator compartment (9) and expels it into the atmosphere through a plurality of expulsion holes (19) in an upper cover (20), is housed in one of them.

Depending on the embodiment, the refrigerator compartment (9) has a plurality of refrigerating perimeter walls (47) that may have irregularities on their surface configured to produce turbulence and facilitate germicidal treatment, made of a transparent material and which, due to their design and arrangement, guide and slow down the volume of flow of the fluid as it passes through same for the necessary and sufficient time, passing it multiple times around the emitters (7) of germicidal radiation.

The germicidal device for air and for liquids also comprises at least one electrostatic system for capturing and retaining particles.

The main motor-turbine-pump (16) is housed in the container plate (8), which also accommodates at least one battery (12) configured to service and charge other external electronic devices such as tablets or mobile phones, and likewise the container plate (8) also houses the electronic boards (13).

Both the primary labyrinth plate (5) and the secondary labyrinth plate (6) have a reflective inner coating configured to reflect and bounce light emissions from the emitters (7) of germicidal radiation.

In the EXHALATION MODE of operation, when the user only requires purifying the exhalation, the germicidal device for air and for liquids is configured so that the air collected through the diffuser (34) coming from the user's exhalation, through a mask (46) configured to adapt to the user's face and collect all the exhaled air, transporting it through the main tube (31), and an encapsulation (35) configured as an anti-particle and anti-microorganism filter, enters the germicidal system forcefully by means of the main motor-turbine-pump (16), entering through an outer opening (21) housed in the container plate (8), then entering through the main motor-turbine-pump (16) through the secondary labyrinth plate (6), which consists of a plurality of channels and secondary perimeter walls (11) that may have irregularities on their surface which are configured to produce turbulence and facilitate germicidal treatment and which, due to their design and arrangement, guide and slow down the volume of flow of air as it passes through same for the necessary and sufficient time, passing it multiple times systematically around the emitters (7) of germicidal radiation so that they neutralise any microorganism that it may contain until entering the primary labyrinth plate (5), which consists of a plurality of channels and primary perimeter walls (10) that may have irregularities on their surface which are configured to produce turbulence and facilitate germicidal treatment and which, due to their design and arrangement, guide and slow down the volume of flow of air as it passes through same for the necessary and sufficient time, passing it multiple times around the emitters (7) of germicidal radiation so that they neutralise any microorganism it may contain.

At the end of the path of the primary labyrinth plate (5), the air enters the intermediate plate (4) and is directed to the plate (3) with several compartments, being filtered in one of them where the main filter (2) and the secondary filter (14) are housed, closing said compartment with an interchangeable auxiliary cover (43), which retains the air in the plate (3), entering the intermediate plate (4) again and from there the refrigerator compartment (9), where the exhaled air refrigerates the emitters (7) of germicidal radiation and entrains the ozone, passing through said refrigerator compartment (9) to enter the upper plate (18) comprising several compartments, where one of them houses the secondary motor-turbine-pump (17), which sucks in the air coming from the refrigerator compartment (9) and expels it into the atmosphere through a plurality of expulsion holes (19) housed in the upper cover (20).

In DUAL MODE of operation, the germicidal device for air and for liquids allows the user to purify the inhaled and exhaled air so that the air collected in the mask (46) coming from the user's exhalation enters the germicidal device forcefully by means of a small secondary motor-turbine-pump (17) through a secondary tube (32) connected to a mask (46), a funnel, a diffuser, a hopper or other means of capturing the fluid and an interchangeable cover (27), being filtered through an auxiliary filter (22), passing to the intermediate plate (4) and passing through the refrigerator compartment (9), until reaching the secondary filter (14) which is housed inside the plate (3), from which the purified exhaled air exits to the outside through the removable, interchangeable cover (1).

The air to be inhaled enters the device naturally or forcefully by means of a main motor-turbine-pump (16), through a removable and interchangeable cover (1), then passing through and being filtered by a main anti-particle and anti-microorganism filter (2), which is housed inside a plate (3) comprising several compartments and a plurality of holes through which the air passes to the intermediate plate (4), which internally accommodates the primary labyrinth plate (5) and the secondary labyrinth plate (6) that surround a plurality of emitters (7) of germicidal radiation that are in turn inside a refrigerator compartment (9) configured to refrigerate the emitters (7) of germicidal radiation and entrain and expel to the outside the possible emissions of ozone produced by said emitters (7) of germicidal radiation. Once the fluid enters the intermediate plate (4), it is then directed towards the primary labyrinth plate (5), which consists of a plurality of channels and primary perimeter walls (10) that may have irregularities on their surface which are configured to produce turbulence and facilitate germicidal treatment, made of a transparent material and which, due to their design and arrangement, guide and slow down the flow volume of flow of the fluid as it passes through same for the necessary and sufficient time, passing it multiple times around the emitters (7) of germicidal radiation. Said emitters (7) of germicidal radiation are configured to emit ultraviolet light, neutralising any microorganism that the fluid may contain, and to emit radiation at any wavelength, not limited to ultraviolet light, emitting radiation of any other type that has germicidal and/or disinfecting power.

At the end of the path of the primary labyrinth plate (5), the fluid enters the secondary labyrinth plate (6), which comprises a plurality of channels and transparent secondary perimeter walls (11) which, due to their design and arrangement, guide and slow down the volume of flow of the fluid as it passes through same for the necessary and sufficient time, passing it multiple times around the emitters (7) of germicidal radiation, and likewise said secondary perimeter walls (11) may have irregularities on their surface which are configured to produce turbulence and facilitate germicidal treatment, and both the primary labyrinthine plate (5) and the secondary labyrinthine plate (6) are separated by means of a separator (42) configured to separate the labyrinthine channels into two differentiated spaces, and it may be transparent.

The air exits through the container plate (8) to reach the encapsulation (35) configured as an anti-pollen filter, the main tube (31) being connected to a mask (46) or a diffuser (34) configured to send the fluid towards the user's mask through a set of accessories explained later, and said tube (31) is also connected to any air diffusion means other than those mentioned, such as a hopper, guide grill, etc., to allow the direct exit of the treated air towards the user's face.

In these embodiments, both at the inlet and outlet of the air around the device, it is possible to render one or more aerosols or products that improve the breathing of people with respiratory difficulties and that purify the air that passes through the device inert.

In LIQUID MODE, the germicidal device for air and for liquids can be used interchangeably to take the water collected in a container, reservoir, river, swamp or others, which does not carry any pressure, entering the device with the help of the main motor-turbine-pump (16) and the secondary motor-turbine-pump (17), and it can also enter through a pressurised line from an external pump. In both cases, the pressure or lack thereof is managed by a pressure reducing solenoid valve (23) that adjusts the volume of flow to enter the device, opening up in the case of no pressure or regulating itself when there is, cutting off power of the main motor-turbine-pump (16) and the secondary motor-turbine-pump (17). The water enters from the outside through an auxiliary tube (33) connected to the container plate (8), passing through a cubicle (24) configured to house biocidal tablets that the user can put in if deemed suitable, the water then passing through a first battery of several water filters (25) configured to eliminate any impurities that the water may contain before entering the device, which is already irradiated by the emitters (7) of germicidal radiation through a transparent panel (26), located between the battery of several water filters (25) and the container plate (8), the water then passing through the pressure reducing solenoid valve (23), where it may or may not then be driven towards the device by the main motor-turbine-pump (16), then entering the secondary labyrinth plate (6) which, by means of the secondary perimeter walls (11) it has, guide the volume of flow of water as it passes through same, passing it multiple times around the emitters (7) of germicidal radiation so that they neutralise any microorganism that the water may contain in its path, until entering the primary labyrinth plate (5) which, due to its design and arrangement, guide and slow down the volume of flow of water as it passes through same the necessary and sufficient time, passing it multiple times around the emitters (7) of germicidal radiation so that they neutralise any microorganisms it may contain. At the end of the path of the primary labyrinth plate (5), the water exits to enter the intermediate plate (4) and is directed to the plate (3) with several compartments, being filtered in one of them, where the main filter (2) and the secondary filter (14) are housed, said compartment of the filter being closed by an interchangeable auxiliary watertight cover (43), which retains the water in the plate (3), entering the intermediate plate (4) again, and from there to the refrigerator compartment (9), where the water refrigerates the emitters (7) of germicidal radiation and entrains the ozone, passing through said refrigerator compartment (9), to enter an upper plate (18) comprising several compartments, where one of them houses the secondary motor-turbine-pump (17), which pumps the water coming from the refrigerator compartment (9), to enter an upper plate (18) comprising several compartments, where one of them houses the secondary motor-turbine-pump (17), which pumps the water coming from said refrigerator compartment (9) then directing it to an auxiliary filter (22), contained by an interchangeable cover (27) configured to be interchangeable with the upper cover (20), the water exiting through the outlet opening (28) to the outside already purified through a secondary tube (32).

In different embodiments, the water inlets and outlets around the device allow one or more products that purify the water that passes through the device to be inserted.

Depending on how the user wants to use the germicidal device for air and for liquids, the device has all the elements of all the previous modes and can be used in combination as MIXED MODE where, if it is only for air, the retention plug (29), installed in the hole (30), must be used and the upper cover (20) or the interchangeable cover (27) must be used, while if it is to be used for water, the retention plug (29) in the outer opening (21) and the interchangeable cover (27) must be used.

The filters comprised in the germicidal device for air and for liquids, such as the main filter (2), the secondary filter (14) and the encapsulation (35), can be activated carbon filters and/or filters made of germicidal technical fabric made up of silver ions.

The entire germicidal device for air and for liquids comprises a set of non-return valves configured to prevent changes in the direction of the fluid inside the device.

The entire germicidal device for air and for liquids comprises a set of safety switches that cut off power in the event that the user opens the device while energised.

The germicidal device for air and for liquids comprises at least one temperature regulation system configured to heat or refrigerate the fluids that flow inside same.

The germicidal device for air and for liquids is made up of several accessories, comprising the main tube (31) that is light and insulating from the external air flow, which is configured to couple a flexible diffuser (34) at the end thereof, where both are traversed by and provided with a band (36) with magnetic properties and configured to be malleable, allowing the user to adapt it ergonomically to their physiognomy, allowing connection and fastening to the user by means of several different shapes, which can be used independently or combined with one another.

In one embodiment, the main tube (31) is made of plastic paper, or is made up of various types of fibres or silver-ion technical fabric and is configured to acquire volume as a result of the positive pressure exerted by the main motor-turbine-pump (16) and the secondary motor-turbine-pump (17).

In another embodiment, the fastening of the main tube (31) to the user is carried out by means of connecting the band (36) in various retentive forms with a connector (37), configured to have on one of the sides or surface thereof adhesive means or an addition of one or more layers of a hypoallergenic foam, and on the base of which it can be preferably and not exclusively connected to a surface (38) of one or more layers of a material that is soft to the touch, foamed with high adhesion to the skin and easy to remove from same, which adheres to the user's skin. Through anchors (39) which are configured to retain through the containment area (50) the elastic bands of a generic mask that the user can wear, to have on one of the sides or surface (51) thereof preferably and not exclusively adhesive means or an addition of one or more layers of hypoallergenic foam and which have at one end a surface (38) that allows it to adhere to the skin.

In another embodiment of the fastening of the anchors (39) to the surface (38), this is carried out by means of a connector (52) that is housed in the gap (53), which confers a rotation that imparts flexibility to the union, cushioning the tension of the rubber bands when the user moves naturally when turning their head, thus improving its usability and versatility, as it further relieves ear pain.

Depending on the embodiment of the fastening of the anchors (39) with the surface (38) through the surface (51), the elements may contain materials in their manufacture that allow magnetic properties instead of having a connector (52), as well as also using a Velcro-type fabric or the like to couple and attach the assembly to the user's head and neck.

In another embodiment, the fastening of the main tube (31) to the user is carried out by means of connecting the band (36) with a magnetic connection (45) that is attached to a strap (40) configured to be housed on the user's neck or head, which has retentive cavities or prominences not depicted, comprising on the inside thereof a malleable sheet (41) that allows ergonomic adaptation to the user's physiognomy. Said strap (40) is retained in the position desired by the user, as a result of the opposite force exerted against it by the elastic bands that make up any generic mask, through the containment area (50) of magnetic supports (44) configured to be connected in any position along the strap (40) and which replace the user's ears as an anchor. Depending on the embodiment, the strap (40) may contain elements in its manufacture that allow magnetic properties instead of having a sheet (41) as well as be made of hypoallergenic rubber or silicone, configured to be mixed in its composition with a ferromagnetic material.

In another embodiment, the fastening of the main tube (31) and the secondary tube (32) to the user's head or neck is carried out by means of a single strap or rubber band.

In another embodiment, the fastening of the main tube (31) to the user is carried out by means of directly connecting the main tube (31) to the mask (46) as well as the direct connecting of the secondary tube (32) to the mask (46).

## Claims

1. A germicidal device for air and for liquids that allows filtering and sterilising both the air to be inhaled and the air exhaled, separately or at the same time, as well as purifying liquids, comprising a main cavernous hollow body of at least
• a primary labyrinth plate (5) comprising a plurality of channels and primary perimeter walls (10) configured to guide and slow down the flow of the fluid and at least
• a refrigerator compartment (9) configured to channel the fluids and mix them with the hot and poisonous gases generated by at least
• an emitter (7) of germicidal radiation configured to irradiate the fluids,
**characterised in that**
• in one operation mode, the fluids that pass through the primary labyrinth plate (5) take different paths and never mix with the fluids that pass through the refrigerator compartment (9);
• in another operation mode, the same fluids that pass through the primary labyrinth plate (5) also pass through the refrigerator compartment (9).

2. The germicidal device for air and for liquids according to claim 1, **characterised in that** it comprises at least one main filter (2) which is configured to filter fluids before entering the inside of the system or exiting same.

3. The germicidal device for air and for liquids according to any of the preceding claims, **characterised in that** it comprises at least one main motor-turbine-pump (16) that drives and/or sucks the fluids through the labyrinth system.

4. The germicidal device for air and for liquids according to any of the preceding claims, **characterised in that** it comprises at least one secondary motor-turbine-pump (17) that drives and/or sucks the fluids through the labyrinth system.

5. The germicidal device for air and for liquids according to any of the preceding claims, **characterised in that** it comprises at least one diffuser (34) and at least one main tube (31) which are configured to send and/or receive the air flow to and/or from the user's mouth, nose and face area.

6. The germicidal device for air and for liquids according to any of the preceding claims, **characterised in that** it comprises at least one cubicle (24) configured to house biocidal tablets.

7. The germicidal device for air and for liquids according to any of the preceding claims, **characterised in that** it comprises a battery of several filters (25) for liquids configured to filter the water before entering the inside of the device.

8. The germicidal device for air and for liquids according to any of the preceding claims, **characterised in that** the battery of water filters (25) are activated carbon filters.

9. The germicidal device for air and for liquids according to any of the preceding claims, **characterised in that** the battery of water filters (25) are filters made of germicidal technical fabric made up of silver ions.

10. The germicidal device for air and for liquids according to any of the preceding claims, **characterised in that** it comprises a transparent panel (26) located between the battery of several water filters (25) and the container plate (8).

11. The germicidal device for air and for liquids according to any of the preceding claims, **characterised in that** it comprises at least one pressure reducing solenoid valve (23) which is configured to regulate the operation of the main motor-turbine-pump (16) and the secondary motor-turbine-pump (17) in the event that it is connected to the water intake of an external pressurised line.

12. The germicidal device for air and for liquids according to any of the preceding claims, **characterised in that** the primary perimeter walls (10) are transparent.

13. The germicidal device for air and for liquids according to any of the preceding claims, **characterised in that** the secondary perimeter walls (11) are transparent.

14. The germicidal device for air and for liquids according to any of the preceding claims, **characterised in that** the main motor-turbine-pump (16) and the secondary motor-turbine-pump (17) are configured to change the flow parameters by tilting the blades and/or their speed to vary the volume of flow of the fluid.

15. The germicidal device for air and for liquids according to any of the preceding claims, **characterised in that** it comprises at least one main filter (2), one secondary filter (14), one auxiliary filter (22) and one encapsulation (35) at the inlet of the air circuits of the entire assembly.

16. The germicidal device for air and for liquids according to any of the preceding claims, **characterised in that** the main filter (2) is an activated carbon filter.

17. The germicidal device for air and for liquids according to any of the preceding claims, **characterised in that** the main filter (2) is a filter made of germicidal technical fabric made up of silver ions.

18. The germicidal device for air and for liquids according to any of the preceding claims, **characterised in that** the secondary filter (14) is an activated carbon filter.

19. The germicidal device for air and for liquids according to any of the preceding claims, **characterised in that** the secondary filter (14) is a filter made of germicidal technical fabric made up of silver ions.

20. The germicidal device for air and for liquids according to any of the preceding claims, **characterised in that** the encapsulation (35) is an activated carbon filter.

21. The germicidal device for air and for liquids according to any of the preceding claims, **characterised in that** the encapsulation (35) is a filter made of germicidal technical fabric made up of silver ions.

22. The germicidal device for air and for liquids according to any of the preceding claims, **characterised in that** it comprises an encapsulation (35) that is located before the air inlet in the device.

23. The germicidal device for air and for liquids according to any of the preceding claims, **characterised in that** the main filter (22) is an activated carbon filter.

24. The germicidal device for air and for liquids according to any of the preceding claims, **characterised in that** the main filter (22) is a filter made of germicidal technical fabric made up of silver ions.

25. The germicidal device for air and for liquids according to any of the preceding claims, **characterised in that** it comprises a secondary filter (22) that is located before the air inlet in the device.

26. The germicidal device for air and for liquids according to any of the preceding claims, **characterised in that** it comprises a main filter (2) that is located before the air inlet in the device.

27. The germicidal device for air and for liquids according to any of the preceding claims, **characterised in that** it comprises at least one power battery (12) for the autonomous operation of the assembly.

28. The germicidal device for air and for liquids according to any of the preceding claims, **characterised in that** the power battery (12) is configured to service and charge other external electronic devices such as tablets or mobile phones.

29. The germicidal device for air and for liquids according to any of the preceding claims, **characterised in that** it comprises at least one electrostatic system for capturing and retaining particles.

30. The germicidal device for air and for liquids according to any of the preceding claims, **characterised in that** it comprises a set of safety switches that cut off power in the event that the user opens the device while energised.

31. The germicidal device for air and for liquids according to any of the preceding claims, **characterised in that** it comprises a set of non-return valves configured to prevent changes in the direction of the fluid inside the system.

32. The germicidal device for air and for liquids according to any of the preceding claims, **characterised in that** the primary perimeter walls (10) have irregularities on their surface which are configured to produce turbulence and facilitate the germicidal treatment of the emitters (7) of germicidal radiation in all the particles of the fluid.

33. The germicidal device for air and for liquids according to any of the preceding claims, **characterised in that** the secondary perimeter walls (11) have irregularities on their surface which are configured to produce turbulence and facilitate the germicidal treatment of the emitters (7) of germicidal radiation in all the particles of the fluid.

34. The germicidal device for air and for liquids according to any of the preceding claims, **characterised in that** between the primary perimeter walls (10) and the secondary perimeter walls (11) there is a separator (42) configured to separate the labyrinth channels into two differentiated spaces.

35. The germicidal device for air and for liquids according to any of the preceding claims, **characterised in that** the separator (42) is transparent.

36. The germicidal device for air and for liquids according to any of the preceding claims, **characterised in that** the primary perimeter walls (10) are transparent.

37. The germicidal device for air and for liquids according to any of the preceding claims, **characterised in that** the secondary perimeter walls (11) are transparent.

38. The germicidal device for air and for liquids according to any of the preceding claims, **characterised in that** the emitters (7) of germicidal radiation emit radiation at any wavelength, not limited to ultraviolet light, emitting radiation of any other type that has germicidal and/or disinfecting power.

39. The germicidal device for air and for liquids according to any of the preceding claims, **characterised in that** it comprises several air inlets around the device configured to insert one or more aerosols or products that improve the breathing of people with respiratory difficulties.

40. The germicidal device for air and for liquids according to any of the preceding claims, **characterised in that** it comprises several air inlets around the device configured to insert one or more aerosols or products that purify the air that passes through the device.

41. The germicidal device for air and for liquids according to any of the preceding claims, **characterised in that** it comprises several air outlets around the device configured to insert one or more aerosols or products that purify the air that passes through the device.

42. The germicidal device for air and for liquids according to any of the preceding claims, **characterised in that** it comprises several water inlets around the device configured to insert one or more products that purify the water that passes through the device.

43. The germicidal device for air and for liquids according to any of the preceding claims, **characterised in that** it comprises several water outlets around the device configured to insert one or more products that purify the water that passes through the device.

44. The germicidal device for air and for liquids according to any of the preceding claims, **characterised in that** it comprises at least one temperature regulation system configured to heat or refrigerate the fluids.

45. The germicidal device for air and for liquids according to any of the preceding claims, **characterised in that** the main tube (31) is made of plastic paper, configured to acquire volume as a result of the positive pressure exerted by the main motor-turbine-pump (16) and the secondary motor-turbine-pump (17).

46. The germicidal device for air and for liquids according to any of the preceding claims, **characterised in that** the main tube (31) is made of various types of fibres or silver-ion technical fabric, configured to acquire volume as a result of the positive pressure exerted by the main motor-turbine-pump (16) and the secondary motor-turbine-pump (17).

47. The germicidal device for air and for liquids according to any of the preceding claims, **characterised in that** the main tube (31) comprises a malleable band (36).

48. The germicidal device for air and for liquids according to any of the preceding claims, **characterised in that** the band (36) is part of the main tube (31).

49. The germicidal device for air and for liquids according to any of the preceding claims, **characterised in that** the primary tube (31) and the secondary tube (32) can be attached to the neck or head of the user by means of a simple rubber band or cord.

50. The germicidal device for air and for liquids according to any of the preceding claims, **characterised in that** it comprises a magnetic connector (37), the base of which is formed by an adhesive surface (51).

51. The germicidal device for air and for liquids according to any of the preceding claims, **characterised in that** the connector (37) has on one of its sides or surface adhesive means or an addition of one or more layers of a hypoallergenic foam surface (38).

52. The germicidal device for air and for liquids according to any of the preceding claims, **characterised in that** it comprises a magnetic connector (37) configured to serve as an intermediate support, to adhere to the skin of the user and to adhere to the band (36) and in turn to the main tube (31).

53. The germicidal device for air and for liquids according to any of the preceding claims, **characterised in that** it comprises anchors (39) that also have an adhesive surface (38) configured to adhere to the skin and allows the user to attach their mask.

54. The germicidal device for air and for liquids according to any of the preceding claims, **characterised in that** the anchors (39) have on one of the sides or surface thereof adhesive means or an addition of one or more layers of a hypoallergenic foam surface (38).

55. The germicidal device for air and for liquids according to any of the preceding claims, **characterised in that** the surface (38) is connected to the anchor (39) through the connector (52), configured to be housed in the cavity (53) that is in the anchor (39).

56. The germicidal device for air and for liquids according to the preceding claim, **characterised in that** the anchor (39) tilts and rotates with respect to the surface (38) by means of the connector (52).

57. The germicidal device for air and for liquids according to any of the preceding claims, **characterised in that** the anchor (39) is connected to the surface (38) by means of magnetic properties, by means of Velcro or the like.

58. The germicidal device for air and for liquids according to any of the preceding claims, **characterised in that** it comprises a magnetic connection (45) configured to be attached to the surface of a strap (40) housed on the neck or head of the user, which has some retentive cavities or prominences, which are comprised inside same by a malleable sheet (41) that allows the ergonomic adaptation to the user's physiognomy.

59. The germicidal device for air and for liquids according to any of the preceding claims, **characterised in that** the strap (40) is retained in the desired position through the traction force of the rubber bands of the mask exerted in the containment area (50) of the magnetic supports (44).

60. The germicidal device for air and for liquids according to any of the preceding claims, **characterised in that** the strap (40) is made of hypoallergenic rubber or silicone, configured to be mixed in its composition with ferromagnetic material.

61. The germicidal device for air and for liquids according to any of the preceding claims, **characterised in that** the strap (40) is made of hypoallergenic rubber or silicone, configured to be traversed on the inside by a sheet (41).

62. The germicidal device for air and for liquids according to any of the preceding claims, **characterised in that** both the primary labyrinth plate (5) and secondary labyrinth plate (6) have a reflective inner coating configured to reflect and bounce light emissions from the emitters (7) of germicidal radiation.

63. The germicidal device for air and for liquids according to any of the preceding claims, **characterised in that** the primary tube (31) is directly connected to a mask (46).

64. The germicidal device for air and for liquids according to any of the preceding claims, **characterised in that** the primary tube (31) and the secondary tube (32) are directly connected to a mask (46).

65. The germicidal device for air and for liquids according to any of the preceding claims, **characterised in that** the secondary tube (32) is connected to a mask (46), a funnel, a diffuser, a hopper or other means of capturing the fluid, to capture the air exhaled by the user.

66. The germicidal device for air and for liquids according to any of the preceding claims, **characterised in that** the secondary tube (32) is connected to a mask (46), a funnel, a diffuser, a hopper or other means of capturing the fluid, to guide the air inhaled by the user.

67. The germicidal device for air and for liquids according to any of the preceding claims, **characterised in that** the refrigeration compartment (9) has a plurality of refrigerating perimeter walls (47) which, due to their design and arrangement, guide and slow down the volume of flow of the fluid as it passes through same, passing it multiple times around the emitters (7) of germicidal radiation.

68. The germicidal device for air and for liquids according to any of the preceding claims, **characterised in that** the refrigerating perimeter walls (47) are transparent.

69. The germicidal device for air and for liquids according to any of the preceding claims, **characterised in that** the refrigerating perimeter walls (47) have irregularities on their surface which are configured to produce turbulence.

70. The germicidal device for air and for liquids according to any of the preceding claims, **characterised in that** the filters (2, 14, 22, 25, 35) are anti-pollen filters.

71. The germicidal device for air and for liquids according to any of the preceding claims, **characterised in that** it mainly comprises at least one retainer (39) and at least one support (44) configured to contain the elastic bands of any mask, by means of a containment area (50) where said bands are housed.

72. The germicidal device for air and for liquids according to any of the preceding claims, **characterised in that** the supports (44) are configured to tilt on the strap (40).

73. The germicidal device for air and for liquids according to any of the preceding claims, **characterised in that** in one operation mode the possible ozone and other gases generated by the emitters (7) of germicidal radiation are discharged from the device safely without being breathed.

74. The germicidal device for air and for liquids according to any of the preceding claims, **characterised in that** in another operation mode the possible ozone and other gases generated by the emitters (7) of germicidal radiation are also used by the device as germicidal agents.

75. The germicidal device for air and for liquids according to any of the preceding claims, **characterised in that** it comprises several operation modes for use in a single device, which can be selected by the user by simply interchanging some components and which are:
• INHALATION MODE: which renders the air to be inhaled by the user inert, using part of the system for this and sending a positive pressure of purified air to the user's mask and face, displacing the exhaled CO2 through same, while the other part of the system is used to refrigerate the emitters (7) of germicidal radiation and discharge ozone and other harmful gases along with heat, preventing them from being breathed;
• EXHALATION MODE: which reverses the paths of the flow and renders the contaminated air exhaled by the user inert by using the entire system which, in addition to refrigerating the emitters (7) of germicidal radiation, said expelled air mixes and sweeps away the ozone, the other gases and the heat generated by said emitters (7) to increase the disinfecting power of the device, also using the germicidal properties of said gases, until all the already purified and clean air exits to the outside;
• DUAL MODE: which changes the paths and flow of the fluid and simultaneously renders the air to be inhaled and the air exhaled by the user inert, where part of the system provides purified air to the user and the other part is dedicated to refrigerating the emitters (7) of germicidal radiation and eliminate the gases and heat they generate while being purified;
• LIQUID MODE: which renders any liquid collected by external pressure, gravity and/or by suction of the motors-turbine (16 and 17) inert, taking advantage of the entire system to refrigerate the emitters (7) of germicidal radiation, sweep away ozone and heat generated and purify the water, which is free of ozone in 30-40 minutes;
• MIXED MODE: which can render liquids and air inert interchangeably by using the labyrinthine paths of the modes described above and interchanging components where, if it is only for air, the retention plug (29) installed in the hole (30) must be used and the upper cover (20) or the interchangeable cover (27) must be used, while if it is to be used for water, the retention plug (29) in the outer opening (21) and the interchangeable cover (27) must be used.
